# EUROPÄISCHE PATENTSCHRIFT

(11) **EP 1 307 238 B1**
(45) Veröffentlichungstag und Bekanntmachung des Hinweises auf die Patenterteilung: **12.09.2007**
(21) Anmeldenummer: 01955363.5
(22) Anmeldetag: 20.07.2001
(51) Int. Cl.: A61K 49/22

(54) **KONTRASTMITTEL BESTEHEND AUS GEOMETRISCH HOCHGEORDNETEN PROTEINEN FÜR DIE MEDIZINISCHE DIAGNOSTIK**
CONTRAST MEDIUM CONSISTING OF HIGHLY REGULAR PROTEINS FOR MEDICAL DIAGNOSTICS
AGENT DE CONTRASTE FORMÉ PAR DES PROTEINES FORTEMENT ORDONNEES POUR LE DIAGNOSTIC MEDICAL

(30) Priorität: 03.08.2000 DE 10037832
(43) Veröffentlichungstag der Anmeldung: 07.05.2003
(73) Patentinhaber: Forschungszentrum Karlsruhe GmbH, 76133 Karlsruhe (DE); Universität Heidelberg, 69117 Heidelberg (DE)
(72) Erfinder: DINJUS, Eckard, 76774 Leimersheim (DE); BAUM, Marina, 07747 Jena (DE); BEHRENS, Silke Dr., 76135 Karlsruhe (DE); UNGER, Eberhard, 07751 Jena-Cospeda (DE); KÜCHERER, Helmut, 68789 St. Leon-Roth (DE); BEKEREDJIAN, Raffi, 69121 Heidelberg (DE); RUEF, Johannes, 69151 Neckargmünd (DE)
(86) Internationale Anmeldenummer: PCT/EP2001/008377
(87) Internationale Veröffentlichungsnummer: WO 2002/011771

(56) Entgegenhaltungen:
- WO-A-98/16257
- DE-A- 19 624 332
- DE-A- 19 631 544
- US-A- 5 124 471
- DOLBNYA, I. P. ET AL: "Combined investigation of nontraditional x-ray contrast agents for indirect lymphography" NUCL. INSTRUM. METHODS PHYS. RES., SECT. A (1995), 359(1,2), 357-60 , XP001079740
- MERTIG M ET AL: "Three-dimensional metallization of microtubules" THIN SOLID FILMS, ELSEVIER-SEQUOIA S.A. LAUSANNE, CH, Bd. 305, Nr. 1-2, 1. August 1997 (1997-08-01), Seiten 248-253, XP004088988 ISSN: 0040-6090 in der Anmeldung erwähnt

## Beschreibung

Die Erfindung betrifft ein Ultraschallkontrastmittel zur Verwendung in der medizinischen Diagnostik gemäß dem ersten Patentanspruch.

Kontrastmittel werden in der Medizin zur Optimierung bildgebender diagnostischer Verfahren eingesetzt.

In der Echokardiographie wird zwischen Rechtsherz- und Linksherz-Kontrastmitteln unterschieden. Rechtsherz-Kontrastmittel passieren aufgrund ihrer Partikelgröße und Instabilität die Lungenkapillaren nicht und sind somit lediglich zur Erkennung von sogenannten Shunts zwischen rechter und linker Herzhälfte und zur Verstärkung von Trikuspidal- und Pulmonalflußgeschwindigkeiten geeignet. Linksherz-Kontrastmittel sind klein und stabil genug, um nach intravenöser Gabe die Lungenkapillaren zu passieren. Bislang untersuchte Anwendungsbereiche sind die Verstärkung von Dopplersignalen (z. B. Pulmonalvenenfluß), die Endokardkonturabgrenzung, z. B. bei der Streßechokardiographie, und die Beurteilung der Myokardperfusion.

Die ersten Ultraschallkontrastmittel mit diesen Eigenschaften bestanden aus luftgefüllten Mikrosphären mit mittleren Durchmessern von ca. 2 bis 5 µm, die von Albumin oder Galactose-Palmitinsäurehüllen umgeben waren. Solche Kontrastmittel werden beispielsweise von Hayat Alkan-Onyuksel et al, Journal of Pharmaceutical Sciences Vol. 85, No. 5 /1996) 486-490 beschrieben.

Die Herstellung der Mikrosphären erfolgt durch hochfrequente Ultraschallanregung von Humanalbumin oder durch die Lösung von Galactose in Wasser. Diese Kontrastmittel haben ähnliche rheologische Eigenschaften wie rote Blutkörperchen und wirken sich daher nicht auf die systemische oder koronare Hämodynamik aus. Die Signalverstärkungsdauer der von Albuminhüllen umgebenen Mikrosphären beträgt jedoch nur wenige Sekunden, bedingt durch die druck abhängige Zerstörung der luftgefüllten Mikrosphären und der Löslichkeit von Luft im Blut. Für länger anhaltende Anfärbungen des linken Ventrikels sind Kontrastmittel mit größerer Stabilität notwendig.

Eine größere Stabilität der Kontrastmittel wird erzielt, wenn die Luft in den Mikrosphären durch ein inertes Gas ersetzt wird (D. M. Skyba et al, Journal of the American College of Cardiology, Vol 28 No. 5.(1996) 1292-1300). Inerte Gase sind in Blut weitaus weniger löslich als Luft. Hierdurch kann die Signalverstärkungsdauer auf bis zu 4 Minuten verlängert werden.

Die Effektivität der Kontrastverstärkung hängt von den physikalischen Eigenschaften der Mikrobläschen, dem erzeugten akustischen Feld und den Signalverarbeitungsverfahren ab. Neuere Verfahren in der Echokardiographie (das sogenannte "Harmonic Imaging") nutzen die Kompressibilität des Kontrastmittels aus, um die reflektierten harmonischen Oberschwingungen zu registrieren und somit eine Verstärkung der Kontrastwirkung zu erzielen. Ein "Harmonic Imaging" ist jedoch bei den üblicherweise verwendeten Ultraschallgeräten nicht möglich.

Echosignalintensitäten im Ultraschallbild werden durch das akustische Rückstreuverhalten der Struktur im Schallfeld bestimmt. So entstehen Streu- und Binnenechos an anatomischen Strukturen, die kleiner sind als die Wellenlänge des einfallenden Ultraschalls. Die Rückstreuintensität ist dabei proportional zur sechsten Potenz des Mikrosphären-Radius.

Es ist bereits vorgeschlagen worden, die beschriebenen Echokontrastmittel mit Antikörpern zu koppeln, um Thromben besser darzustellen (Lanza et al, Circulation Vol. 94, No. 12 (1996) 3334-3339). Hierzu wurde ein eigens dazu entwickeltes Kontrastmittel (Perfluorcarbon-Phosphatidylethanolamin- Mikroemulsion) zunächst biotinyliert und über eine Biotin-Avidin-Bindung unter Verwendung von anti-Fibrinogen-Antikörpern spezifisch an die Oberfläche des Thrombus gebunden.

Die Kopplung von Antikörpern über eine direkte kovalente Bindung an das Kontrastmittel wird von S. M. Demos et al, Journal of the American College of Cardiology Vol. 33, No. 3 (1999) 867-875 beschrieben.

Von geringerem Nutzen sind die bekannten, an Antikörper gekoppelten Kontrastmittel vor allem wegen ihrer geringen Stabilität und damit ihrer geringen Lebensdauer in vivo. Die Kontrastwirkung ist deshalb nach einer Injektion des Kontrastmittels zeitlich eng begrenzt.

Aus Dolbnya et al: "Combined Investigation of nontraditional x-ray contrast agents for indirect lymphography", Nucl. Instrum. Methods Phys. Res., Sect. A (1995) 359 (1,2), 357-60, XP001079740 ist bekannt, dass mit kolloidalem Gold besetztes Albumin als nichtraditionelles Röntgen-Kontrastmittel verwendet werden kann. Spezifische Reagentien für verschiedene Krankheiten können erhalten werden, wie z. B. markierte Antitumor Antikörper oder markierte Toxine.

Die US 5,124,471 beschreibt bifunktionale Cyclohexal DTPA Liganden. Solche Liganden sind nützlich zum Radiolabeln von Proteinen. Als Beispiele für Proteine zur Verwendung in MetallChelat-Protein-Konjugaten werden u. a. Antikörper, Antigene und Blutproteine angegeben.

Die WO98/16257 befasst sich mit Ultraschallkontrastmitteln, die aus stabilisierten Mikrosphären bestehen, deren proteinartige Hüllen mit Metallsalzlösungen behandelt wurden. Zu den bevorzugten Metallen gehört u. a. Titan. Die Mikropartikel können targetspezifisch sein. Zur Ultraschallstabilität werden in-vitro und in-vivo Angaben gemacht.

Die DE 196 31 544 A betrifft die Verwendung von Metall-Clustern als Kontrastmittel in der Diagnostik und in der Strahlentherapie. Metalle der Ordnungszahlen 20-32, 38-51 oder 56-83 kommen in der NMR-, Röntgen- und Radiodiagnostik zum Einsatz. Die Oberflächenmodifikation erfolgt z. B. durch physiologisch verträgliche Mittel wie Pollysaccharide.

Der Erfindung liegt daher die Aufgabe zugrunde, die beschriebenen Nachteile zu vermeiden und insbesondere ein Kontrastmittel für medizinische Zwecke vorzuschlagen, das in vivo eine wesentlich höhere Stabilität aufweist.

Die Aufgabe wird durch das im ersten Patentanspruch beschriebene Kontrastmittel gelöst.

Erfindungsgemäß wird ein Ultraschallkontrastmittel vorgeschlagen, das aus Mikrotubuli besteht und mit Metallclustern besetzt ist. Die Mikrotubuli sind mit Metallclustern besetzt. Die Metallcluster können aus einem körperverträglichen Metall, insbesondere einem Edelmetall, beispielsweise aus Gold, aber auch aus Silber, Palladium, Platin oder Titan und deren Legierungen bestehen. Die Größe der Metallcluster liegt im Bereich von 2 nm bis 100 nm, wobei Clustergrößen von 5 nm bis 20 nm besonders bevorzugt werden. Die Clusterdichte wird vorzugsweise so hoch wie technisch erreichbar gewählt.

Derartige mit Metallclustern besetzte hochgeordneten Proteine werden in einem völlig anderen technischen Zusammenhang in der WO 97/448837 und in der Veröffentlichung von R. Kirsch et al.: "Three-dimensional metallization of microtubules", Thin Solid Films 305 (1997) 248-253 beschrieben. In der erstgenannten Druckschrift bilden die mit Metallclustern besetzten Proteine eine Zwischenstufe bei der Herstellung von mikromechanischen und mikroelektrömechanischen Bauteilen. In beiden Druckschriften wird die Herstellung der clusterbesetzten Proteine und deren Struktur eingehend beschrieben.

Es hat sich gezeigt, dass mit diesen mit Metallclustern besetzten Mikrotubuli ein wirksames Ultraschalljontrastmittel für die medizinische Diagnostik, insbesondere für die Echokardiographie und die Computertomographie, geschaffen wird.

Das erfindungsgemäße Kontrastmittel kann venös, arteriell oder über einen Katheter appliziert werden.

Gegenüber den bekannten Kontrastmitteln ergeben sich die folgenden Vorteile.

In Vorversuchen wurde bereits eine erheblich größere Stabilität dieser Kontrastmittel in einem Ultraschallfeld nachgewiesen, so daß die mit der geringen Stabilität bekannter Kontrastmittel verbundenen Nachteile vermieden werden können. Der Kontrasteffekt beruht erfindungsgemäß nicht auf eingeschlossenen Gasblasen, sondern auf fest auf den Proteinen haftenden Metallclustern.

Die clusterbesetzten hochgeordneten Proteine lassen sich zudem in an sich bekannter Weise mit Antikörpern koppeln. Dies kann entweder über bispezifische Antikörper oder über eine direkte kovalente Bindung über funktionelle Gruppen erfolgen. Als bispezifische Antikörper können solche Antikörper eingesetzt werden, welche auf einer Seite eine anti-Tubulin-Komponente besitzen und auf der anderen Seite gegen das zu untersuchende Epitop gerichtet sind. Alternativ können sogenannte "Linker" eingesetzt werden, die mittels kovalenter Bindungen das clusterbesetzte Protein und den Antikörper verbinden. Die erheblich höhere Stabilität der clusterbesetzten Proteine macht solche Kontrastmittel erst attraktiv. Als Antikörper kommen praktisch alle an sich bekannten, monoklonalen oder polyklonalen Antikörper'in Betracht. Mögliche Epitope sind Fibrinogen oder Gp IIb/IIIa z. B. für Thromben-Untersuchungen, Kollagen, der Willebrand Faktor-Rezeptor, VCAM-1 oder ICAM-1 z. B. für Endothel-Untersuchungen; die jeweiligen Antikörper entsprechen den Epitopen: Anti-Fibrinogen-Antikörper, Anti-Gp IIb/IIIa-Antikörper etc.

Da die clusterbesetzten Proteine im Vergleich zu gasgefüllten Mikrosphären eine sehr geringe Kompressibilität aufweisen, sind die aufwendigen Maßnahmen im Rahmen des "Harmonic Imaging" zur Erkennung der harmonischen Oberschwingungen des Kontrastmittels nicht notwendig.

Die Erfindung wird im folgenden anhand von Ausführungsbeispielen und Figuren näher erläutert.

Es zeigen
Fig. 1 mit 10 nm großen Gold-Clustern besetzte Mikrotubuli;
Fig. 2 Palladium-Cluster auf einem Mikrotubulus;
Fig. 3 fluoreszierende mit Gold-Clustern besetzte Mikrotubuli;
Fig. 4 sonographische Beispiele für in-vitro-Untersuchungen zur Echogenität clusterbesetzter *Mikrotubuli.*

### Beispiel 1

### Darstellung von Mikrotubuli mit Gold- und Palladium-Clustern

a) Assemblierung und Fixierung von Mikrotubuli
   Die Assemblierung von Mikrotubuli aus einer Proteinlösung mit einer Endkonzentration von 1 mg/ml Tubulin erfolgte in einer Pufferlösung I von 20 mM Piperazindiethansulfonsäure (PIPES) mit pKₐ 6,8 und 80 mM NaCl, 0,5 mM MgCl₂ sowie 1 mM Ethylenglykol-bis-(2-Aminoethyl)-Tetraessigsäure (EGTA) durch Zugabe von 10 mM Guanosintriphosphat (GTP) und 10 µM Taxol bei einer Temperatur von 37°C in 20 Minuten. Die Mikrotubuli wurden zunächst in 0,05 %, dann nach 1 h in 1 % Glutaraldehyd unter starkem Rühren fixiert. Die Reduktion der freien Aldehydgruppen erfolgte mit einer wäßrigen NaBH₄-Lösung (50 mg NaBH₄ in 1 ml Wasser). Nach ca. 30 min wurde die Reaktionslösung gegen 2000 ml Wasser/Puffer I im Verhältnis von 10:1 über Nacht dialysiert.
b) Herstellung von mit Gold-Clustern besetzten Mikrotubuli 100 ml einer kolloidalen Goldsuspension (Polyscience: Au-Partikeldurchmesser 10 nm, Au-Konzentration 44 µg/ml) wurden in der Zentrifuge auf ca. 10 ml aufkonzentriert, mit 20 ml der nach Beispiel 1a assemblierten und fixierten Mikrotubuli versetzt und innerhalb von 30 min bei Raumtemperatur auf den Mikrotubuli immobilisiert. Das Reaktionsgemisch wurde im Dialyseschlauch gegen Polyethylenglykol (20000) auf ca. 4 ml aufkonzentriert. Nach dem Aufkonzentrieren betrug die Goldkonzentration ca. 1,1 mg/ml und die' Proteinkonzentration ca. 5 mg/ml. Die mittlere Clustergröße ergab sich zu 10 nm.

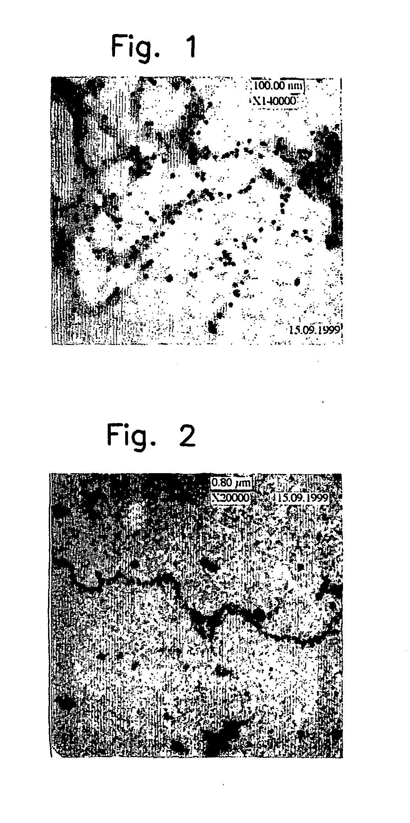

   In Fig. 1 ist eine Rasterelektronenmikroskop-Aufnahme mit 140000-facher Vergrößerung dieser aufkonzentrierten Lösung dargestellt. Die Mikrotubuli sind als vernetzte, vor dem helleren Hintergrund dunkel erscheinende Fäden und die Goldcluster als schwarze Punkte abgebildet.
c) Darstellung von mit Palladium-Clustern besetzten Mikrotubuli 20 ml der nach Beispiel 1a assemblierten und fixierten Mikrotubuli mit einer Proteinkonzentration von 1 mg/ml wurden mit 80 ml einer wäßrigen Na₂PdCl₄-Lösung (0,0125 M) versetzt und bei 90°C in 1 h mit 8 ml einer wäßrigen Lösung des Trinatriumsalzes der Zitronensäure (0,25 M) reduziert. Die Probe wurde im Dialyseschlauch auf ca. 4 ml gegen Polyethylen (20000) aufkonzentriert. Nach dem Aufkonzentrieren betrug die Pd-Konzentration ca. 26.6 mg/ml und die Protein-Konzentration ca. 5 mg/ml. Die Clustergröße lag bei ca. 2 nm.

In Fig. 2 ist eine Rasterelektronenmikroskop-Aufnahme mit 20000-facher Vergrößerung dieser aufkonzentrierten Lösung dargestellt.

### Beispiel 2

### Kopplung von Antikörpern

Die Kopplung von Antikörpern an die mit Metallclustern besetzten Mikrotubuli wurde durch die Kopplung eines Primäräntikörpers (Anti-β-Tubulin) an die Proteinstruktur und anschließende Kopplung eines fluoreszenz(Cy3)-markierten Sekundärantikörpers demonstriert.

Dazu wurden zunächst die verwendeten Objektträger 10 min mit einer wäßrigen Polydiallyldimethylammoniumchlorid (PDDA)-Lösung (1 %) behandelt, 5 min unter laufendem Wasser von überschüssigem PDDA befreit und dann getrocknet. 75 µl der nach Beispiel 1a und 1b hergestellten Proben wurden 30 min auf dem beschichteten Objektträger inkubiert und mehrmals mit je 75 µl Waschlösung I (Phosphatpuffer: pH 7,2, 8 g NaCl, 0,2 g KCl, 1,15 g Na₂HPO₄•2. H₂O, 0,2 g KH₂PO₄, 1 % Bovinserumalbumin (BSA)) gewaschen. Danach wurde die Probe mit je 75 µl Primärantikörper (Anti-β-Tubulin (50 µg/ml), monoclonal antibody, äntimouse IgG der Firma Böhringer Mannheim, 1:50 verdünnt mit Waschlösung I) 1 h in einer feuchten Kammer bei Raumtemperatur inkubiert. Nach wiederholtem Waschen mit Waschlösung I wurde die Probe mit einem Cy3 markierten Sekundärantikörper (Antimous IgG (1 mg/ml in 0,01 M Phosphatpuffer, 1% BSA, 15 mM NaN₃) der Fa. Sigma, 1 : 300 verdünnt mit Waschlösung I, Absorption (Cy3) : 552 nm, Emission (Cy3) : 565 nm) für 1 h in der Dunkelheit in einer feuchten Kammer bei Raumtemperatur inkubiert. Nach wiederholtem Waschen mit Waschlösung I wurde dann 1 Tropfen Citifluor in Glycerol und PBS-Lösung auf den Objektträger gegeben. Zur Kontrolle wurde außerdem eine Probe ohne Zugabe von Primärantikörper hergestellt. Die Fluoreszenz-Untersuchungen der Probe erfolgten im Mikroskop (Fluoreszenz-Anregung: λ = 540 - 560 nm).

Die Untersuchung der Negativkontrolle (ohne Primärantikörper) zeigte keine Fluoreszenz. Bei allen anderen Proben (reine Mikrotubuli und Mikrotubuli mit Au-Clustern) fluoreszierten die markierten Mikrotubuli. Somit ist die Kopplung von Antikörpern an die mit Metallclustern besetzten Mikrotubuli möglich.

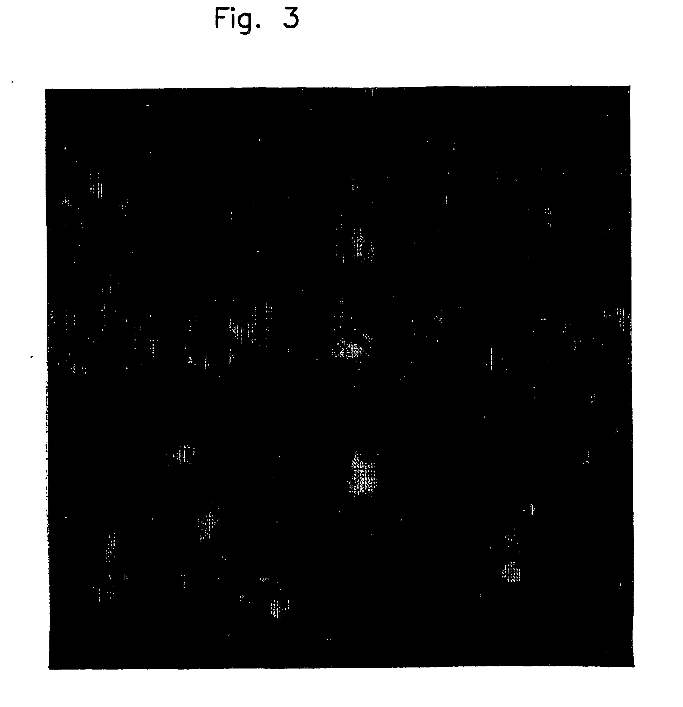

Fig. 3 zeigt die fluoreszierenden, mit Au-Clustern beladenen Mikrotubuli.

### Beispiel 3

### Ultraschallversuche mit clusterbesetzten Mikrotubuli

Zur Durchführung von Ultraschallversüchen mit metallclusterbesetzten Mikrotubuli wurden in Testreihen Latex-Ballons mit verschiedenen Mikrotubuli-Suspensionen gefüllt und in einem Wasserbad durch eine 2,5 MHz Echokardiographiesonde im B-mode untersucht. Zum Vergleich wurde ein Kontrollballon mit reiner Pufferlösung (Puffer 20 mM Piperazindiethansulfonsäure (PIPES), pKₐ 6,8, 80 mM NaCl, 0,5 mM MgCl₂, 1 mM Ethylenglykol-bis-(2-Aminoethyl)-Tetraessigsäure (EGTA), 4 ml) sonographisch aufgezeichnet.

In einen mit 10 ml Puffer gefüllten Latexballon wurde eine Probe assemblierter und fixierter Mikrotubuli (Protein-Konzentration ca. 3 mg/ml) injiziert. Weiterhin wurde je ein Latexballon mit 1 ml Pufferlösung und 3 ml der nach Beispiel 1a und 1b hergestellten Proben gefüllt.

Während die reine Mikrotubuliprobe keinen eindeutigen und reproduzierbaren Kontratrasteffekt im Ultraschall verursachte, zeigte sich bei den mit Goldclustern beladenen Mikrotubuli ein deutlicher Kontrasteffekt.

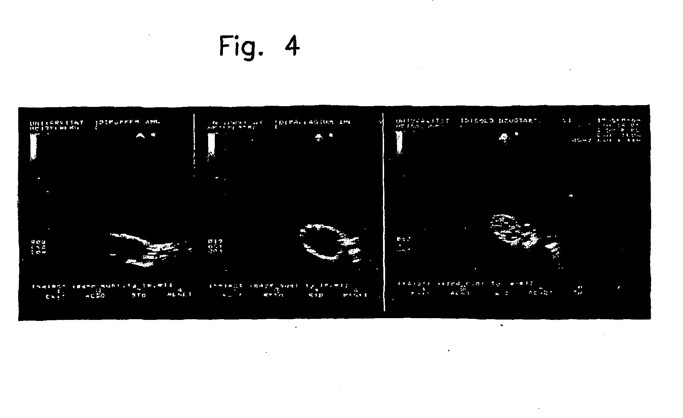

In Fig. 4 ist der Unterschied in der Echogenität zwischen reinen, metallfreien Mikrotubuli und goldbeladenen Mikrotubuli dargestellt. Es sind jeweis die Ultraschallbilder von Latexballons (4 ml Inhalt) in einem Wasserbad abgebildet. Links: Kontrolle mit Puffer - keine Kontrastwirkung; rechts: mit Goldclustern beladene Mikrotubuli - Kontrastwirkung.

Die Suspension mit den goldbeladenen Mikrotubuli wurde mit Puffer verdünnt. Die verdünnten Suspensionen wurden nach der Ultra-schalluntersuchung videodensitometrisch ausgewertet (Tabelle 1). Dabei zeigte sich erwartungsgemäß bei zunehmender Verdünnung eine Abnahme der Kontrastintensität.

### Tabelle 1:

Videodensitometrische Untersuchung der sonographischen Aufnahmen von Verdünnungen der goldbeladenen Mikrotubulin-Suspension. Aufgeführt sind die Grauwerte der unverdünnten (4 ml) sowie der mit. 5, 10 und 20 ml verdünnten Mikrotubuli-Suspensionen. Zum Vergleich sind die entsprechenden Daten einer Kontrolluntersuchung mit NaCl- und einer Levovist®-Lösung aüfgeführt, sowie die unverdünnte goldbeladene Mikrotubuli-Suspension im "Harmonic Imaging" Modus. (Levovist ist Linksherzkontrastmittel bestehend aus luftgefüllten Mikrosphären mit einer Hülle von Galaktose/Palmitinsäure und einem Durchmesser von ca. 2 µm. Die Viskosität wird mit 1, 5 - 4 cP (25°C) und die Osmolalität mit ca. 950 (37°C) angegeben.)

**Tabelle 1:**

| **Substanz** | **Grauwertintensität (Mittelwert)±SD** |
|---|---|
| Au-MT (unverdünnt) | 141 ± 35 |
| Au-MT (mit 5 ml Puffer verdünnt) | 133 ± 49 |
| Au-MT (mit 10 ml Puffer verdünnt) | 84 ± 64 |
| Au-MT (mit 20 ml Puffer verdünnt) | 42 ± 55 |
| Levovist® | 180 ± 35 (mit Harmonic Imaging) |
| NaCl | 0,1 ± 0, 7 |

| | |
|---|---|
| MT: Mikrotubuli SD: Standard-Abweichung | |

## Patentansprüche

1. Ultraschallkontrastmittel zur Verwendung in der medizinischen Diagnostik bestehend aus Mikrotubuli, die mit Metallclustern einer Größe von 2 bis 100 nm besetzt sind.

2. Ultraschallkontrastmittel nach Anspruch 1, bei dem als Metall für die Cluster ein Edelmetall, Chrom, Nickel oder Titan eingesetzt wird.

3. Ultraschallkontrastmittel nach Anspruch 2 mit Metallclustern aus Gold.

4. Ultraschallkontrastmittel nach einem der Ansprüche 1 bis 3, bei dem die Proteine an Antikörper angekoppelt sind, die sich an Strukturen eines zu untersuchenden Epitops ankoppeln.

5. Ultraschallkontrastmittel gemäß einem der Ansprüche 1 bis 3 zur Verwendung in der Echokardiographie.

6. Ultraschallkontrastmittel gemäß einem der Ansprüche 1 bis 3 zur Verwendung in der Computertomographie.

## Claims

1. Ultrasonic contrast medium for use in medical diagnosis, consisting of microtubuli covered with metal clusters of 2 to 100 nm in size.

2. Ultrasonic contrast medium according to Claim 1, with the metal of the clusters being a noble metal, chromium, nickel, or titanium.

3. Ultrasonic contrast medium according to Claim 2, with the metal clusters being made of gold.

4. Ultrasonic contrast medium according to one of Claims 1 through 3, with proteins being coupled to antibodies that attach to the structures of an epitope to be examined.

5. Ultrasonic contrast medium according to one of Claims 1 through 3 for use in echo cardiography.

6. Ultrasonic contrast medium according to one of Claims 1 through 3 for use in computer tomography.

## Revendications

1. Agent de contraste ultrasonore destiné à être utilisé dans le diagnostic médical et constitué de microtubules revêtues de clusters métalliques d'une taille allant de 2 à 100 nm.

2. Agent de contraste ultrasonore selon la revendication 1 dans lequel le métal mis en oeuvre dans les clusters est un métal noble, du chrome, du nickel ou du titane.

3. Agent de contraste ultrasonore selon la revendication 2 comprenant des clusters métallique en or.

4. Agent de contraste ultrasonore selon l'une quelconque des revendications 1 à 3 dans lequel les protéines sont liés à des anticorps couplant sur des structures d'un épitope à examiner.

5. Agent de contraste selon l'une quelconque des revendications 1 à 3 destiné à être utilisé dans l'échocardiographie.

6. Agent de contraste selon l'une quelconque des revendications 1 à 3 destiné à être utilisé dans la scanographie.
